# EUROPÄISCHE PATENTSCHRIFT

(11) **EP 1 305 109 B1**
(45) Veröffentlichungstag und Bekanntmachung des Hinweises auf die Patenterteilung: **11.08.2004**
(21) Anmeldenummer: 01969563.4
(22) Anmeldetag: 01.08.2001
(51) Int. Cl.: B01J 13/02, B01J 13/10, B01J 13/22

(54) **POLYELEKTROLYTKAPSELHERSTELLUNG DURCH OBERFLÄCHENPRÄZIPITATION**
PRODUCTION OF POLYELECTROLYTE CAPSULES BY SURFACE PRECIPITATION
PRODUCTION DE CAPSULES DE POLYELECTROLYTE PAR PRECIPITATION SUPERFICIELLE

(30) Priorität: 02.08.2000 DE 10037707; 11.10.2000 DE 10050382
(43) Veröffentlichungstag der Anmeldung: 02.05.2003
(73) Patentinhaber: Max-Planck-Gesellschaft zur Förderung der Wissenschaften e.V., 80539 München (DE)
(72) Erfinder: VOIGT, Andreas, 13051 Berlin (DE); SUKHORUKOV, Gleb, 14471 Potsdam (DE); RADTCHENKO, Igor, Yaroslavl, 152935 (RU); ANTIPOV, Alexei, 28 Moskau 113054 (RU); DONATH, Edwin, 16845 Giesenhorst (DE); MÖHWALD, Helmuth, 55411 Bingen (DE)
(74) Vertreter: Dey, Michael, Dipl.-Chem. Dr.
(86) Internationale Anmeldenummer: PCT/EP2001/008909
(87) Internationale Veröffentlichungsnummer: WO 2002/009865

(56) Entgegenhaltungen:
- EP-A- 0 972 563
- EP-A- 1 116 516
- WO-A-01/64330
- WO-A-99/47253

## Beschreibung

Die Erfindung betrifft ein Verfahren zur Herstellung von Nano- bzw. Mikrokapseln, die eine Polyelektrolythülle umfassen, durch Oberflächenpräzipitation aus der Lösung.

DE 198 12 083.4, DE 199 07 552.2, EP 98 113 181.6 und WO 99/47252 offenbaren ein Verfahren zur Herstellung von mit einer Polyelektrolythülle beschichteten Kapseln durch schichtweisesAufbringen von Polyelektrolyten auf Templatpartikel. Ein Vorteil dieses Verfahrens gegenüber früheren Verfahren zur Herstellung von Mikrokapseln besteht darin, dass monodisperse Kapseln mit definiert eingestellter Wandstärke hergestellt werden können. Aus wirtschaftlichen Gesichtspunkten ist jedoch problematisch, dass der schichtweise Aufbau einer Kapselhülle zeit- und arbeitsaufwendig sein kann.

Buchhammer und Lunkwitz (Ber. Bunsenges. Phys. Chem. 100 (1996), 1039-1044) und Oertel et al. (Coll. Surf. 57 (1991), 375-381) beschreiben die Oberflächenmodifikation von organischen und anorganischen Partikeln durch Ablagerung eines Komplexes aus positiv und negativ geladenen Polyelektrolyten auf der Partikeloberfläche. Ein Nachteil dieses Verfahrens ist, dass die resultierenden Schichten eine geringe Stabilität aufweisen.

Eine Aufgabe der Erfindung bestand deshalb darin, ein neues Verfahren zur Herstellung von Kapseln mit hoher Stabilität und Hüllen geringer Wandstärke bereitzustellen, bei dem die Nachteile des Standes der Technik mindestens teilweise beseitigt sind.

Diese Aufgabe wird gelöst durch ein Verfahren zum Aufbringen einer Hülle auf Templatpartikel mittels Oberflächenpräzipitation aus einer Lösung, wobei dieses Verfahren die Schritte umfasst:
(a) Bereitstellen einer Dispersion von Templatpartikeln geeigneter Größe in einer salzhaltigen Flüssigphase, die zur Bildung der Hülle erforderliche Komponenten in gelöster Form enthält, und
(b) Präzipitieren der Komponenten aus der Flüssigphase auf die Templatpartikel unter solchen Bedingungen, dass eine Hülle mit einer Dicke von 1 bis 100 nm um die Templatpartikel erzeugt wird,
wobei die zur Bildung der Hülle erforderlichen Komponenten
(i) zwei entgegengesetzt geladene Polyelektrolyten,
(ii) ein polyvalentes niedermolekulares Kation und einen negativ geladenen Polyelektrolyten oder
(iii) ein polyvalentes niedermolekulares Anion und einen positiv geladenen Polyelektrolyten
umfassen.

Überraschenderweise wurde festgestellt, dass bei Beschichtung von Templatpartikeln durch Oberflächenpräzipitation aus einer salzhaltigen Lösung Kapseln mit einer definierten und geringen Hüllendicke und mit selektiv steuerbaren Permeabilitätseigenschaften erhalten werden können. Dabei können verschiedene Arten von Hüllen, z.B. Polyelektrolythüllen oder Polyelektrolyt/lon-Hüllen erzeugt werden.

Die in der Flüssigkeit gelösten Salze tragen wesentlich zur Stabilität der durch Präzipitation gebildeten Hüllen bei. Beispiele für geeignete Salze sind alle wasserlöslichen niedermolekularen Salze, darunter anorganische Salze, wie Chloride, Bromide, Nitrate, Sulfate und Carbonate ein- und mehrwertiger Alkali-, Erdalkalimetalle oder Übergangsmetalle wie Eisen, Silber, Kupfer. Die Konzentrationen liegen vorzugsweise im Bereich von 0,5 mM bis 1 M oder höher in den Fällen, in denen die Wirkung des Salzes in der Minderung der elektrostatischen Wechselwirkungen zwischen Polyelektrolyten einerseits und den Polyelektrolyten und den Templatoberflächen andererseits besteht. Werden spezifische Wechselwirkung und/oder Komplexierung mehrwertiger niedermolekularer Anionen und Kationen mit den Polyelektrolyten erforderlich um verteilte Polyelektrolytpools in der Hüllflüssigkeit zu erzeugen, befinden sich die Konzentrationen der Salze vorzugsweise im Bereich von 0,001 bis 10 mM.

Das erfindungsgemäße Verkapselungsverfahren ermöglicht die Verkapselung von beliebigen kolloidalen Partikeln. Neben festen Partikeln können auch flüssige Partikel, z.B. emulgierte Öltröpfchen oder flüssig-kristalline Partikel, oder gasförmige Partikel, z.B. Luft- oder andere Gasbläschen beschichtet werden. Die Größe von zu verkapselnden Flüssigkeits- oder Gaspartikeln kann z.B. durch Zugabe von grenzflächenaktiven Stoffen zur Flüssigphase eingestellt werden.

Als feste Templatpartikel können beliebige kolloidale Feststoffe, insbesondere anorganische Materialien, z.B. Metalle, Keramiken, Oxide oder Salzkristalle, organische Materialien wie Polymerlatizes, organische Präzipitate, verfestigte Öltröpfchen, Gele oder Kristalle, Melaminformaldehydpartikel, Lipidvesikef, biologische Templatpartikel wie Zellen oder Pollen eingesetzt werden. Die Größe der Templatpartikel kann - insbesondere bei Verwendung biologischer Templatmaterialien - bis zu 50 µm erreichen. Vorzugsweise ist die Größe der Templatpartikel jedoch bis zu 10 µm, besonders bevorzugt von 5 nm bis 10 µm und am meisten bevorzugt von 5 nm bis 5 µm. Die Form der Templatpartikel ist nicht kritisch. Sowohl sphärische als auch anisotrope Partikel können beschichtet werden.

In einer bevorzugten Ausführungsform werden Templatpartikel verkapselt, die einen Wirkstoff enthalten, z.B. selbst einen Wirkstoff darstellen. Dieser Wirkstoff kann beispielsweise ausgewählt werden aus Katalysatoren, insbesondere Enzymen, z.B. Enzymkristallen, Nanopartikeln, z.B. magnetischen Nanopartikeln, biologischen Makromolekülen etc., pharmazeutischen Wirkstoffen, Sensormolekülen, z.B. radioaktiven oder nicht-radioaktiven Markierungsmolekülen wie etwa Fluoreszenzmarkierungen, Kristallen, Polymeren und Gasen. Die Wirkstoffpartikel können der Flüssigphase zugesetzt werden oder darin durch Präzipitation erzeugt werden. Die Präzipitation kann vor oder/und während der Kapselbildung erfolgen und zu Kristallen oder/und amorphen Strukturen führen.

Beispielsweise können die Kapseln zum Einbringen von organischen Flüssigkeiten wie etwa Alkoholen oder Kohlenwasserstoffen, z.B. Hexanol, Octanol, Octan oder Decan, oder zum Verkapseln von Gasen für Ultraschallkontrastmittel verwendet werden. Solche mit einer organischen, nicht mit Wasser mischbaren Flüssigkeit gefüllten Kapseln können auch für chemische Reaktionen, z. B. Polymerisationsreaktionen eingesetzt werden. So kann das Monomer über dessen Verteilungsgleichgewicht gezielt im Innenraum der Kapseln angereichert werden. Gegebenenfalls kann die Monomerenlösung bereits vor Beginn der Synthese im Innenraum eingekapselt werden.

Es können jedoch auch Wirkstoffe verkapselt werden, die aufgrund ihrer Größe nicht die Polyelektrolythülle durchdringen können. Hierzu wird der einzuschließende Wirkstoff an das Templatpartikel gekoppelt bzw. immobilisiert oder vom Templatpartikel eingekapselt oder aufgenommen, z.B. durch Phagozytose oder Endozytose bei lebenden Zellen oder durch Verkapselung von Nanopartikeln in lösliche Templatmaterialien. Nach Desintegration der Templatpartikel wird der Wirkstoff ins Innere der Polyelektrolythülle freigesetzt. Dabei werden zweckmäßigerweise die Bedingungen bei der Desintegration des Templatpartikels so gewählt, dass keine unerwünschte Zersetzung des Wirkstoffs eintritt.

Eine Kopplung des Wirkstoffs an das Templat kann direkt erfolgen, aber auch durch einen Bindevermittler bewirkt werden. Als Bindevermittler werden bevorzugt Moleküle verwendet, die bei bestimmten Bedingungen degradierbar oder abbaubar sind. Besonders bevorzugt wird als Bindevermittler Polymilchsäure verwendet. Hierzu wird der Wirkstoff mittels des Bindevermittlers, insbesondere Polymilchsäure, an das Templatpartikel, beispielsweise ein teilvernetztes Melaminformaldehydpartikel immobilisiert. Auf diese Weise wird der einzuschließende Wirkstoff selbst Bestandteil des Schichtaufbaus bei der Beschichtung des Kerns. Nach der Auflösung der Templatpartikel und gegebenenfalls Degradation der Bindemoleküle wird der Wirkstoff ins Innere der Hülle freigesetzt. Mit diesem Verfahren können beliebige Wirkstoffe in die Hülle eingeschlossen werden, insbesondere Nanopartikel und nicht-biologische makromolekulare Komponenten und bevorzugt biologische Makromoleküle, wie etwa Proteine, insbesondere Enzyme.

Weiterhin können z.B. mit 4-Pyrensulfat (4-PS) kationische Polymere oder Partikel in der Hülle fixiert werden. Durch Herauslösen von 4-PS in Salzlösungen werden diese Partikel dann in das Innere der Hülle freigesetzt.

Die Inkorporation von Wirkstoffen in den von den Hüllen umschlossenen Innenraum kann durch vorherige Einbringung der Wirkstoffe in die Templatpartikel bei Verwendung von reversiblen Mikrogelen als Templatpartikel durchgeführt werden. So ermöglicht beispielsweise die Verwendung von teilvernetzten Methylolmelaminkernen vor der Beschichtung, in gequollene Kerne Substanzen zu inkorporieren, die nach einer reversiblen Schrumpfung im Kern eingeschlossen sind.

In einer weiteren bevorzugten Ausführungsform des erfindungsgemäßen Verfahrens können lösliche Partikel als Templatpartikel verwendet werden. Diese löslichen Partikel lassen sich ohne Zerstörung der um die Partikel herum durch Präzipitation gebildeten Hülle zumindest teilweise desintegrieren. Beispiele für lösliche Partikel sind teilvernetzte Melaminformaldehydpartikel, die durch Einstellung des pH-Werts in dem die umhüllten Partikel enthaltenden Medium auf einen sauren Wert, z.B. ≤ 1,5, aufgelöst werden können, während die Hüllschicht selbst zumindest teilweise intakt bleibt. Teilvernetzte Melaminformaldehydpartikel können auch durch chemische Reaktionen, insbesondere durch Sulfonierung in wässrigen Medien aufgelöst werden. Die Herstellung solcher teilvernetzter Metaminformaldehydpartikel ist ausführlich in WO 99/47252 beschrieben. Weitere Beispiele für auflösbare Templatpartikel sind lösliche Polymerkerne, z.B. Harnstoff-Formaldehyd-Partikel oder Salzkristalle, oder Salzkristalle, z.B. Carbonatverbindungen, deren wässrige Löslichkeit steuerbar ist, oder nicht in Wasser aber in Ethanol lösliche organische Verbindungen, z.B. Cyaninfarbstoffe.

Außerdem können als Templatmaterialien beispielsweise Zellen, z.B. eukaryontische Zellen, wie etwa Säugererythrozyten oder Pflanzenzellen, einzellige Organismen wie etwa Hefen, Bakterienzellen wie etwa E.coli Zellen, Zellaggregate, subzelluläre Partikel wie etwa Zellorganellen, Pollen, Membranpräparationen oder Zellkerne oder durch chemische oder/und biologische Verfahren erzeugte hohle Zellwand- oder Pollenwandpräparate, Viruspartikel und Aggregate von Biomolekülen, z.B. Proteinaggregate wie etwa Immunkomplexe, kondensierte Nukleinsäuren, Ligand-Rezeptor-Komplexe etc. verwendet werden. Das erfindungsgemäße Verfahren eignet sich auch zur Verkapselung lebender biologischer Zellen und Organismen. Ebenso als Template geeignet sind Aggregate amphiphiler Materialien, insbesondere Membranstrukturen wie etwa Vesikel, z.B. Liposomen oder Micellen sowie andere Lipidaggregate.

Die Desintegration biologischer Templatpartikel kann durch Zugabe von Lysereagenzien erfolgen. Dabei sind Lysereagenzien geeignet, die biologische Materialien wie Proteine oder/und Lipide auflösen können. Vorzugsweise enthalten die Lysereagenzien ein Deproteinisierungsmittel, beispielsweise Peroxoverbindungen wie etwa H₂O₂ oder/und Hypochloritverbindungen wie etwa Natrium- oder Kaliumhypochlorit. Überraschenderweise erfolgt die Desintegration der Templatpartikel innerhalb einer kurzen Inkubationsdauer, z.B. 1 min bis 1 h bei Raumtemperatur. Die Desintegration der Templatpartikel ist weitgehend vollständig, da selbst bei elektronenmikroskopischer Betrachtung der verbleibenden Hüllen keine Reste der Partikel mehr nachweisbar sind. Bei Einbau biologischer Materialien in die Hülle können Kapseln mit teilaufgelösten Hüllen erzeugt werden.

Die bei der Desintegration der Templatpartikel gebildeten Fragmente, z.B. im Fall von teilvernetzten Melaminformaledhypartikeln, die bei Auflösung entstandenen Oligomere, können durch Poren, insbesondere Nanoporen, der Hüllwand aus dem Inneren der Kapseln nach außen austreten. Anschließend können sie - sofern gewünscht - von den Kapseln abgetrennt werden. Diese Abtrennung kann durch dem Fachmann bekannte Verfahren durchgeführt werden, z.B. durch Dialyse, Filtration, Zentrifugation oder/und kontrollierte Phasenseparation abgetrennt. Eine Abtrennung von Templatpartikelfragmenten ist oftmals jedoch nicht notwendig. Die Kapseln können auch ohne Abtrennungsschritt verwendet werden.

Darüber hinaus können auch flüssige oder gasförmige Templatpartikel verwendet werden, z.B. Tropfen einer Mikro- oder Miniemulsion oder Gasblasen entsprechender Größe. Besonders bevorzugt werden als flüssige Templatpartikel Öltropfen verwendet, die durch Ultraschall in einer wässrigen saizhaftigen Lösung emulgiert werden können. Die Größe der Flüssigkeitströpfchen bzw. Gasbläschen lässt sich durch entsprechende Maßnahmen, z.B. Leistung und Dauer einer Ultraschallbehandlung, auf die gewünschten Größen einstellen. Bei dieser Ausführungsform des erfindungsgemäßen Verfahrens können beispielsweise flüssige Wirkstoffe wie etwa Parfümöle, pharmazeutisch wirksame Öle, in Ölen gelöste lipophile feste Wirkstoffe oder Gasbläschen als Kontrastmittel verkapselt werden.

Mit dem erfindungsgemäßen Verfahren ist es auch möglich, Kapseln zum Einschluss von Wirkstoffen herzustellen. Die Beladung des Innenraums mit Molekülen kann dadurch erfolgen, dass die Permeabilität der Hülle als Funktion der externen physikalischen und chemischen Parameter variiert wird. Zur Beladung wird ein Zustand hoher Permeabilität eingestellt. Das eingeschlossene Material wird anschließend durch Veränderung der äußeren Parameter oder/und Verschluss der Poren, beispielsweise durch Kondensation der Hülle oder chemische oder/und thermische Modifikation der Poren oder Kanäle zurückgehalten.

Das erfindungsgemäße Präzipitationsverfahren erlaubt die Abscheidung geladener und nicht geladener Komponenten auf dem Templatpartikel. Die zur Bildung der Hülle erforderlichen Komponenten der Erfindung enthalten zumindest einen Polyelektrolyten, beispielsweise zwei entgegengesetzt geladene Polyelektrolyten oder/und ein polyvalentes Metallkation und einen negativ geladenen Polyelektrolyten.

Unter Polyelektrolyten werden allgemein Polymere mit ionisch dissoziierbaren Gruppen, die Bestandteil oder Substituent der Polymerkette sein können, verstanden. Üblicherweise ist die Zahl dieser ionisch dissoziierbaren Gruppen in Polyelektrolyten so groß, dass die Polymeren in der dissoziierten Form (auch Polyionen genannt) wasserlöslich sind. Hierin werden unter dem Begriff Polyelektrolyte auch lonomere verstanden, bei denen die Konzentration der ionischen Gruppen für eine Wasserlöslichkeit nicht ausreichend sind, die jedoch genügend Ladungen aufweisen, um eine Selbstassemblierung einzugehen. Bevorzugt umfasst die Hülle "echte" Polyelektrolyte. Je nach Art der dissoziierbaren Gruppen werden Polyelektrolyte in Polysäuren und Polybasen unterteilt. Aus Polysäuren entstehen bei der Dissoziation unter Abspaltung von Protonen Polyanionen, die sowohl anorganische als auch organische Polymere sein können.

Polybasen enthalten Gruppen, die in der Lage sind, Protonen, z.B. durch Reaktion mit Säuren unter Salzbildung, aufzunehmen. Polybasen können ketten - bzw. seitenständige dissoziierbare Gruppen aufweisen und bilden durch Aufnahme von Protonen Polykationen.

Erfindungsgemäß geeignete Polyelektrolyte sind sowohl Biopolymere, wie etwa Alginsäure, Gummi Arabicum, Nukleinsäuren, Pektine, Proteine und andere, sowie chemisch modifizierte Biopolymere, wie etwa ionische oder ionisierbare Polysaccharide, z.B. Carboxymethylcellulose, Chitosan und Chitosansulfat, Ligninsulfonate sowie synthetische Polymere, wie etwa Polymethacrylsäure, Polyvinylsulfonsäure, Polyvinylphosphonsäure und Polyethylenimin.

Geeignete Polyanionen umfassen natürlich vorkommende Polyanionen und synthetische Polyanionen. Beispiele für natürlich vorkommende Polyanionen sind Alginat, Carboxymethylamylose, Carboxymethylcellulose, Carboxymethyldextran, Carageenan, Cellulosesulfat, Chondroitinsulfat, Chitosansulfat, Dextransulfat, Gummi Arabicum, Gummi Guar, Gummi Gellan, Heparin, Hyaluronsäure, Pektin, Xanthan und Proteine bei einem entsprechenden pH-Wert. Beispiele für synthetische Polyanionen sind Polyacrylate (Salze der Polyacrylsäure), Anionen von Polyaminosäuren und deren Copolymeren, Polymaleinat, Polymethacrylat, Polystyrolsulfat, Polystyrolsulfonat, Polyvinylphosphat, Polyvinylphosphonat, Polyvinylsulfat, Polyacrylamidmethylpropansulfonat, Polylactat, Poly(butadien/maleinat), Poly(ethylen/maleinat), Poly(ethacrylat/acrylat) und Poly(glycerinmethacrylat).

Geeignete Polybasen umfassen natürlich vorkommende Polykationen und synthetische Polykationen. Beispiele für geeignete natürlich vorkommende Polykationen sind Chitosan, modifizierte Dextrane, z.B. Diethylaminoethylmodifizierte Dextrane, Hydroxymethylcellulosetrimethylamin, Lysozym, Polylysin, Protaminsulfat, Hydroxyethylcellulosetrimethylamin und Proteine bei entsprechenden pH-Wert. Beispiele für synthetische Polykationen sind Polyallylamin, Polyallylaminhydrochlorid, Polyamine, Polyvinylbenzyltrimethylammoniumchlorid, Polybren, Polydiallyldimethylammoniumchlorid, Polyethylenimin, Polyimidazolin, Polyvinylamin, Polyvinylpyridin, Poly(acrylamid/methacryloxypropyltrimethylammoniumbromid), Poly(diallyfdimethylammoniumchlorid/N-lisopropylacrylamid), Poly(dimethylaminoethylacrylat/acrylamid), Polydimethylaminoethylmethacrylat, Polydimethylaminoepichlorhydrin, Polyethyleniminoepichlorhydrin, Polymethacryloxyethyltrimethylammoniumbromid, Hydroxypropylmethacryloxyethyldimethylammoniumchlorid, Poly(methyldiethylaminoethylmethacrylat/acrylamid), Poly(methyl/guanidin), Polymethylvinylpyridiniumbromid, Poly(vinylpyrrolidon/dimethylaminoethylmethacrylat) und Polyvinylmethylpyridiniumbromid.

Es können lineare oder verzweigte Polyelektrolyte eingesetzt werden. Die Verwendung verzweigter Polyelektrolyte führt zu weniger kompakten Polyelektrolytmultifilmen mit einem höheren Grad der Wandporosität. Zur Erhöhung der Kapselstabilität können Polyelektrolytmoleküle innerhalb oder/und zwischen den einzelnen Schichten vernetzt werden, z.B. durch Crosslinking von Aminogruppen mit Aldehyden. Weiterhin können amphiphile Polyelektrolyte, z. B. amphiphile Block- oder Randomcopolymere mit partiellem Polyelektrolytcharakter zur Verringerung der Permeabilität gegenüber polaren kleinen Molekülen eingesetzt werden. Solche amphiphilen Copolymere bestehen aus Einheiten unterschiedlicher Funktionalität, z.B. einerseits sauren oder basischen Einheiten und andererseits hydrophoben Einheiten wie Styrolen, Dienen oder Siloxanen etc. die als Blöcke oder statistisch verteilt über das Polymer angeordnet sein können. Durch Verwendung von Copolymeren, die als Funktion äußerer Bedingungen ihre Struktur ändern, können die Kapselwände bezüglich ihrer Permeabilität oder anderer Eigenschaften definiert gesteuert werden. Hierzu bieten sich beispielsweise schwache Polyelektrolyte, Polyampholyte oder Copolymere mit einem Poly(Nisopropyl-acrylamid)-Anteil, z.B. Poly(N-isopropylacrylamid-acrylsäure) an, die über das Gleichgewicht von Wasserstoffbrückenbindungen ihre Wasserlöslichkeit als Funktion der Temperatur ändern, was mit einer Quellung einhergeht.

Durch Verwendung von unter bestimmten Bedingungen abbaubaren, z.B. photo-, säure-, base-, salz- oder thermolabilen Polyelektrolyten kann über die Auflösung der Kapselwände die Freisetzung von eingeschlossenen Wirkstoffen gesteuert werden. Weiterhin können für bestimmte Anwendungsmöglichkeiten auch leitende Polyelektrolyte oder Polyelektrolyte mit optisch aktiven Gruppen als Kapselkomponenten verwendet werden.

Durch geeignete Wahl der Polyelektrolyte ist es möglich, die Eigenschaften und Zusammensetzung der Polyelektrolythülle der erfindungsgemäßen Kapseln definiert einzustellen. Dabei kann die Zusammensetzung der Hüllen durch die Wahl der Substanzen beim Schichtaufbau in weiten Grenzen variiert werden. Grundsätzlich ergeben sich keine Einschränkungen hinsichtlich der zu verwendenden Polyelektrolyte bzw. lonomere, solange die verwendeten Moleküle eine genügend hohe Ladung aufweisen oder/und die Fähigkeit besitzen, über andere Wechselwirkungsarten, wie beispielsweise WasserstoffbrückenbindungenundloderhydrophobeWechselwirkungen, eine Bindung mit der darunter liegenden Schicht einzugehen.

Geeignete Polyelektrolyte sind somit sowohl niedermolekulare Polyelektrolyte bzw. Polyionen als auch makromolekulare Polyelektrolyte, beispielsweise Polyelektrolyte biologischer Herkunft.

Von besonderer Bedeutung für die Verwendung der Kapseln ist die Permeabilität der Hüllwand. Wie bereits oben ausgeführt, ermöglicht die Vielzahl der zur Verfügung stehenden Polyelektrolyte die Herstellung einer Vielzahl von Hüllkompositionen mit unterschiedlichen Eigenschaften. Insbesondere kann die elektrische Ladung der Außenhülle dem Anwendungszweck angepasst werden. Zudem kann die Innenhülle an jeweils verkapselte Wirkstoffe angepasst werden, wodurch z.B. eine Stabilisierung des Wirkstoffs erzielt werden kann. Daneben kann auch die Permeabilität der Hüllwand durch die Wahl der Polyelektrolyte in der Hülle und durch die Wanddicke sowie die Umgebungsbedingungen beeinflusst werden. Dadurch ist eine selektive Gestaltung der Permeabilistätseigenschaften sowie eine definierte Veränderung dieser Eigenschaften möglich.

Die Permeabilitätseigenschaften der Hülle können durch Poren in mindestens einer der Polyelektrolytschichten weiter modifiziert werden. Solche Poren können bei geeigneter Wahl durch die Polyelektrolyte selbst gebildet werden. Neben den Polyelektrolyten kann die Hülle aber auch andere Substanzen umfassen, um eine gewünschte Permeabilität zu erzielen. So kann insbesondere durch Einbringen von Nanopartikeln mit anionischen oder/und kationischen Gruppen oder von grenzflächenaktiven Substanzen, wie etwa Tensiden oder/und Lipiden, die Permeabilität für polare Komponenten gesenkt werden. Durch die Inkorporation von selektiven Transportsystemen, wie z.B. Carriern oder Kanälen, in die Polyelektrolythülle, insbesondere in Lipidschichten, isteine genaue Anpassung der transversalen Transporteigenschaften der Hülle an den jeweiligen Anwendungszweck möglich. Die Poren oder Kanäle der Hüllwand können durch chemische Modifizierung oder/und Änderung der Umgebungsbedingungen gezielt geöffnet bzw. verschlossen werden. So führt beispielsweise eine hohe Salzkonzentration des Umgebungsmediums zu einer hohen Durchlässigkeit der Hüllwand.

Eine erste Ausführungsform des erfindungsgemäßen Verfahrens umfasst eine komplexe Präzipitation oder Koazervation zwei entgegengesetzt geladener Polyelektrolyte aus alkalischer Lösung, in der beide simultan, ohne miteinander zu reagieren in Lösung gehalten werden. Zu dieser Lösung werden die zu beschichtenden Templatpartikel zugegeben. Anschließend wird mit Säure, z.B. HCl bis in den Neutralbereich titriert, wobei eine Einkapselung der Templatpartikel stattfindet. Nach Abtrennung der eingekapselten Partikel von den Komplexen in der freien Lösung, z.B. durch Filtration, Zentrifugation oder Sedimentation, können die Templatpartikel gegebenenfalls aufgelöst werden.

In einer weiteren bevorzugten Ausführungsform kann die Oberflächenpräzipitation aus einer Lösung enthaltend einen Komplex aus einem niedermolekularen lon und einem entgegengesetzt geladenen Polyelektrolyten erfolgen. Beispiele für geeignete niedermolekulare lonen sind Metallkationen, anorganische Anionen wie Sulfat, Carbonat, Phosphat, Nitrat etc., geladene Tenside, geladene Lipide und geladene Oligomere in Kombination mit einem entsprechend entgegengesetzt geladenen Polyelektrolyten. Hierbei wird eine verteilte Quelle für den einen Polyelektrolyten bei gleichzeitiger Anwesenheit des anderen Polyelektrolyten erzeugt. Der Polyelektrolyt des Komplexes kann sowohl das Polykation als auch das Polyanion sein. Die Wahl hängt von dem vorgelegten Templatpartikel und anderen Vorgaben ab. In dieser Ausführungsform wird beispielsweise ein positiv geladener Polyelektrolyt mit einem mehrfach negativ geladenen niedermolekularen Anion, z.B. Sulfat zu einer Lösung des negativ geladenen Polyelektrolyten und einer Suspension der Templatpartikel gegeben, wobei eine Beschichtung der Templatpartikel stattfindet. Die beschichteten Templatpartikel können von den freien Komplexen beispielsweise durch Zentrifugation, Filtration und anschließendes Waschen abgetrennt werden und - sofern es sich um lösliche Partikel handelt - zur Herstellung von Mikrokapseln aufgelöst werden.

Noch eine weitere bevorzugte Ausführungsform umfasst die Oberflächenpräzipitation aus einer Lösung enthaltend partiell destabilisierte Polyelektrolytkomplexe (Polykation/Polyanion) mittels Salzzugabe oder/und pH-Variation. Hierbei erfolgt eine allmähliche Übertragung von Polyelektrolyten aus den Komplexen auf die Templatoberfläche. Hierzu können der negativ und der positiv geladene Polyelektrolyt in eine wässrige Lösung mit hohem Salzgehalt, vorzugsweise einem Salzgehaltvon ≥ 0,5 Mol/l, z.B. 1 M NaCl, eingebracht und gerührt werden. Nach Zugabe der Templatpartikel werden diese beschichtet. Die beschichteten Templatpartikel können beispielsweise durch Zentrifugation oder Filtration und anschließendes Waschen gewonnen und gegebenenfalls zur Erzeugung von Mikrokapseln aufgelöst werden.

In noch einer weiteren bevorzugten Ausführungsform umfasst die Hülle Metallkationen und mindestens einen negativ geladenen Polyelektrolyten. Als Metallkationen kommen beispielsweise divalente Metallkationen und insbesondere trivalente Metallkationen zum Einsatz. Beispiele für geeignete Metallkationen sind Erdalkalimetalikationen, Übergangsmetallkationen und Seltenerdelementkationen, wie etwa Ca²⁺, Mg²⁺, Y³⁺, Tb³⁺ und Fe³⁺.

Andererseits können auch monovalente Kationen wie Ag⁺ eingesetzt werden. Durch Reduktion der Metallkationen können mit einer Metallschicht überzogene Templatpartikel erzeugt werden.

In noch einer weiteren bevorzugten Ausführungsform umfassen die zur Bildung der Hülle erforderlichen Komponenten zumindest ein Makromolekül, z.B. ein abiogenes Makromolekül, wie etwa ein organisches Polymer, oder ein Biomolekül, wie etwa eine Nukleinsäure, z.B. DNA, RNA oder ein Nukleinsäureanalogon, ein Polypeptid, ein Glykoprotein oder ein Polysaccharid mit einem Molekulargewicht von vorzugsweise ≥ 5 kD, und besonders bevorzugt ≥ 10 kD. Die Makromoleküle können Ladungen tragen, z.B. wie Nukleinsäuren oder aber auch ungeladen sein, wie etwa Polysaccharide, z. B. Dextran. Die Makromoleküle können gegebenenfalls mit Polyelektrolyten oder/und polyvalenten Metallkationen kombiniert werden, wobei z.B. Kombinationen von makromolekularen und niedermolekularen biologischen Zellsubstanzen, makromolekularen und niedermolekularen abiogenen Substanzen und makromolekularen und biogenen und abiogenen Substanzen verwendet werden können.

In noch einer weiteren bevorzugten Ausführungsform umfassen die zur Bildung der Hülle vorgegebenen Komponenten ein Gemisch mehrerer Polyelektrolyte oder/und Lipide oder/und Proteine oder/und Peptide oder/und Nukleinsäuren oder/und weiterer organischer und anorganischer Verbindungen biogener oder abiogener Herkunft. Durch geeignete Zusammensetzung des Lösungsmittels bezüglich Salzgehalt, pH-Wert, Co-Lösungsmittel, Tenside und durch geeignete Wahl der Beschichtungsbedingungen, z.B. Temperatur, rheologische Bedingungen, Anwesenheit elektrischer oder/und magnetischer Felder, Anwesenheit von Licht, werden die diversen Hüllkomponenten zur Selbstassemblierung auf den Templaten veranlasst unter Bildung komplexer Strukturen mit vielfältigen biomimetischen Eigenschaften.

Noch eine weitere bevorzugte Ausführungsform des Verfahrens ist dadurch gekennzeichnet, dass das Zusammenbringen der flüssigen salzhaltigen Hüllphase mit den Templaten die Systembedingungen derart verändert, dass ohne weitere äußere Stimulation mit Ausnahme der permanenten Durchmischung spontan der Aufbau von Hüllen erfolgt, die nach gegebenenfalls erfolger Auflösung der Template intakt bleiben.

Das Präzipitieren gemäß Schritt (b) des erfindungsgemäßen Verfahrens erfolgt unter Bedingungen, sodass um das Templat eine Hülle mit definierter Dicke im Bereich von 1 bis 100 nm, vorzugsweise 1 bis 50 nm, besonders bevorzugt 5 bis 30 nm und am meisten bevorzugt 10 bis 20 nm gebildet wird. Die Wandstärke und die Homogenität der Kapselhülle werden durch die Geschwindigkeit der Polymerpräzipitation bestimmt. Diese hängt im Wesentlichen von der Konzentration der Templatpartikel, der Konzentration der Beschichtungskomponenten und der Geschwindigkeit der die Präzipitation bewirkenden Löslichkeitsveränderung in der Flüssigkeitsphase ab.

Das Präzipitieren kann beispielsweise dadurch erfolgen, dass ein Teil der die Hülle bildenden Komponenten in der Flüssigphase vorgelegt und anschließend eine oder mehrere weitere Hüllkomponenten zugegeben wird. Ein derartiger Präzipitationsschritt kann beispielsweise für eine Kombination von Metallkationen und entgegengesetzt geladenen Polyelektrolyten eingesetzt werden. Eine andere Möglichkeit der Präzipitation besteht darin, dass die zur Bildung der Hülle erforderlichen Komponenten bereits vollständig in der Flüssigphase vorliegen und eine die Präzipitation bewirkende Veränderung der Flüssigphase erfolgt. Diese Veränderung der Flüssigphase kann beispielsweise eine Veränderung des pH-Werts und/öder eine Veränderung der Zusammensetzung der Flüssigphase, z.B. durch Zugabe einer Lösungsmittelkomponente oder/und Entfernen einer Lösungsmittelkomponente umfassen. So kann beispielsweise eine Präzipitation von hydrophilen Biopolymeren wie DNA oder Polysacchariden durch Zugabe von Ethanol zu einer wäßrigen Flüssigphase bewirkt werden, während die Präzipitation von Polyelektrolytkombinationen durch Abdampfen eines organischen Lösungsmittels, wie etwa Aceton aus der Flüssigphase erfolgen kann.

Weiterhin kann das erfindungsgemäße Beschichtungsverfahren die Durchführung von zumindest einem zusätzlichen Beschichtungsschritt vor oder/und nach dem Präzipitationsschritt umfassen. Ein derartiger zusätzlicher Beschichtungsschritt kann beispielsweise das Aufbringen einer oder mehrerer Lipidschichten oder/und das schichtweise Aufbringen von Polyelektrolyten umfassen.

Durch Abscheidung von Lipidschichten oder/und amphiphiler Polyelektrolyten auf der Polyelektrolythülle kann eine Modifizierung der Permeabilität einer Hülle erreicht werden. Auf diese Weise kann die Permeabilität der Hüllen für kleine und polare Moleküle sehr stark vermindert werden. Beispiele für Lipide, die auf den Hüllen abgeschieden werden können, sind Lipide, die mindestens eine ionische oder ionisierbare Gruppe tragen, z.B. Phospholipide wie etwa Dipalmitoylphosphatidinsäure oder zwitterionische Phospholipide wie etwa Dipalmitoylphosphatidylcholin oder auch Fettsäuren bzw. entsprechende langkettige Alkylsulfonsäuren. Bei Verwendung zwitterionischer Lipide können Lipidmultischichten auf der Hülle abgeschieden werden.

Das schichtweise Aufbringen von Polyelektrolyten kann beispielsweise wie in WO 99/47252 beschrieben, erfolgen. Der schichtweise Hüllenaufbau kann mit dem erfindungsgemäßen Präzipitationsschritt beispielsweise so kombiniert werden, dass zunächst auf dem Templatpartikel ein schichtweiser Aufbau von einer geringen Anzahl, z.B. 1 bis 4 Schichten von Polyelektrolyten erfolgt, dem sich ein erfindungsgemäßer Präzipitationsschritt anschließt. Alternativ oder zusätzlich kann auch nach den Präzipitationsschritten eine schichtweise Abscheidung von Polyelektrolyten auf der Hülle erfolgen.

Durch das erfindungsgemäße Verfahren lassen sich monodisperse Kapseln herstellen. So ist es möglich, eine Zusammensetzung mit einer Kapselverteilung zu erhalten, bei der der Anteil an Kapseln, deren Abweichung vom mittleren Durchmesser > 50 % ist, weniger als 20 %, bevorzugt weniger als 10 % und besonders bevorzugt weniger als 1 %.

Die Kapseln sind sehr stabil gegenüber chemischen, biologischen, mechanischen und thermischen Belastungen. Die Kapseln können gegebenenfalls mit eingeschlossenen Wirkstoffen ohne Beeinträchtigung ihrer Eigenschaften getrocknet, eingefroren oder/und gefriergetrocknet werden. Nach dem Auftauen bzw. Resuspendieren in einem Lösungsmittel, z.B. wässrige Lösung, werden unter geeigneten Medienbedingungen oder/und bei entsprechender Medienzusammensetzung wieder intakte Kapseln erhalten.

Bei Trocknung oder Gefriertrocknung der Kapseln wird eine pulverförmige Zusammensetzung erhalten, die in geeigneten Lösungsmitteln, insbesondere in wässrigen Lösungen resuspendiert werden kann. Die Trocknung kann nach bekannten Verfahren durchgeführt werden, insbesondere bei erhöhter oder verringerter Temperatur oder/und reduziertem Druck.

Weiterhin soll die Erfindung durch die nachfolgenden Figuren und Beispiele erläutert werden.
Figur 1 zeigt eine Ausführungsform des erfindungsgemäßen Verfahrens umfassend die einstufige Bildung einer Polyelektrolyt/lon-Hülle auf kolloidalen Templatpartikeln.
Figur 2 zeigt eine weitere Ausführungsform des erfindungsgemäßen Verfahrens, umfassend eine Selbstassemblierung von Polymerfilmen auf der Oberfläche von kolloidalen Partikeln.
Figur 3 zeigt ein rastermikroskopisches konfokales Laserbild von Mikrokapseln, hergestellt durch eine einstufige Präzipitation aus dem ternären Gemisch Wasser/Aceton/Natriumbromid mit PSS⁵⁰⁰ und PBVTAC. Das Templat war ein auflösbares Melaminformaldehydlatexpartikel mit 5,2 µm Durchmesser. Das Lösungsfenster wurde durch Acetonverdampfung verlassen.
Figur 4 zeigt ein rastermikroskopisches konfokales Laserbild von Mikrokapseln, erhalten durch ein einstufiges Verfahren aus dem ternären Gemisch Wasser/Aceton/Natriumbromid mit PSS⁵⁰⁰ und PVBTAC. Das Templat war ein auflösbares Melaminformaldehydlatexteilchen mit 5,2 µm Durchmesser. Das Lösungsfenster wurde durch Zugabe von Wasser verlassen.
Figur 5 zeigt ein konfokales mikroskopisches Bild von kolloidalen Partikeln, beschichtet durch auf fluoreszenzmarkierten PSS und Tb-lonen.
Figur 6 zeigt ein mikroskopisches konfokales Bild von Kolloidpartikeln, beschichtet durch Präzipitate von fluoreszenzmarkiertem Dextran (a) und fluoreszenzmarkierter DNA (b) auf Melaminformaldehydteilchen durch tropfenweise Zugabe von Ethanol zu einer wässrigen Suspension.
Figur 7 zeigt leere Hüllen aus dem Polyanion/Metall-Komplex PSS/Tb, charakterisiert mittels Rasterkraftmikroskopie. In Figur 7a ist die Draufsicht auf eine Kapsel aus 20 Hüllschichten dargestellt und in Figur 7b die Draufsicht auf mehrere Kapseln aus jeweils etwa 100 Hüllschichten.

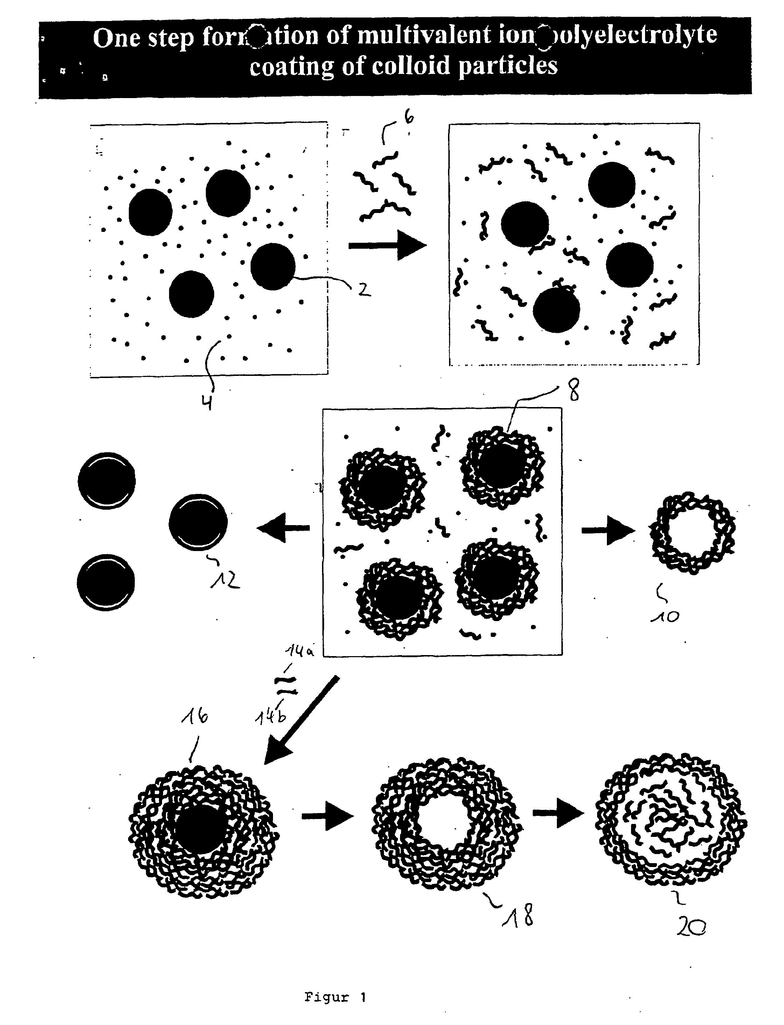

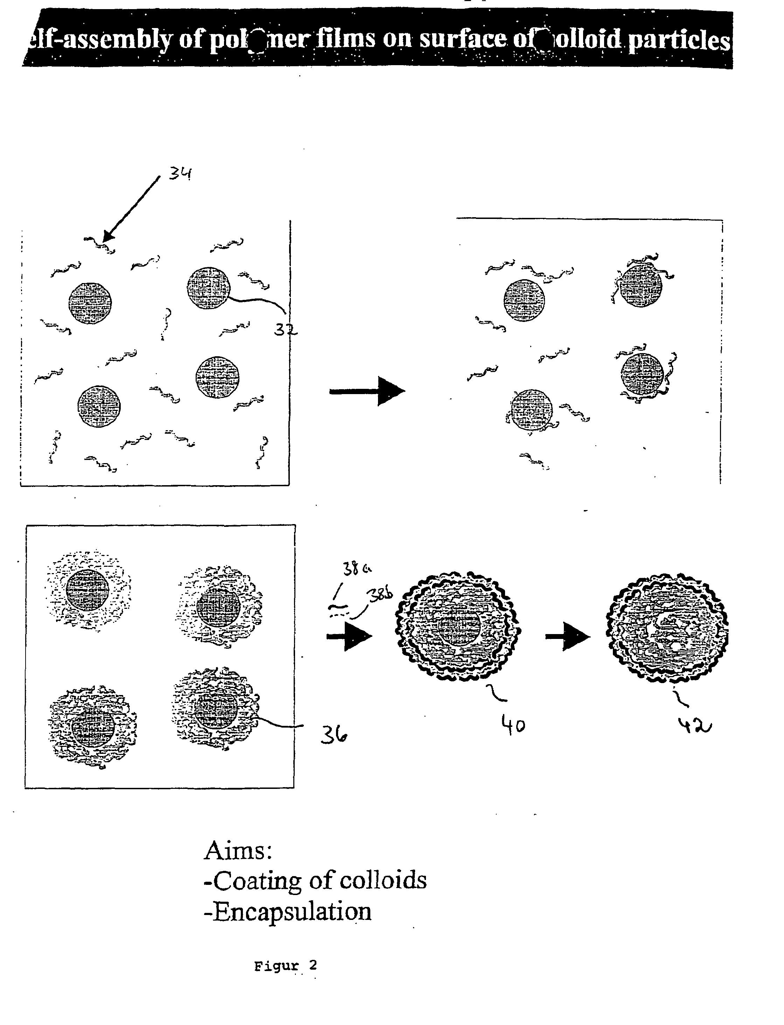

Die Figuren 1 und 2 zeigen eine schematische Darstellung von zwei Ausführungsformen des erfindungsgemäßen Verfahrens. Gemäß Figur 1 wird eine Suspension von Templatpartikeln (2) hergestellt, die Metallionen, z.B. lonen eines polyvalenten Metalles oder Ionen eines Edelmetalles, wie etwa Ag⁺ (4) enthält. Durch tropfenweise Zugabe einer Lösung mit negativ geladenen Polyelektrolytmolekülen (6) erfolgt eine Präzipitation einer lon/Polyelektrolyt-Hülle auf den Templatpartikeln. Die beschichteten Templatpartikel (8) können auf unterschiedliche Art und Weise weiter prozessiert werden. So können durch Auflösung der Templatpartikel leere Kapseln (10) erzeugt werden. Durch Reduktion der Metallionen werden metallbeschichtete Kapseln (12) erhalten. Durch schichtweises Aufbringen entgegengesetzt geladener Polyelektrolyte (14a, 14b) werden Kapseln mit einer anisotropen Hülle hergestellt, wobei der innere Teil eine lon/Polyelektrolyt-Hülle und der äußere Teil eine durch schichtweisen Aufbau erzeugte Polyelektrolyt/Polyelektrolythülle ist. Durch Auflösung der Templatpartikel können anschließend leere Kapseln (18) erzeugt werden. Durch Entfernung der Metallionen (4) kann der innere lon/Polyelektrolyt-Teil der Hülle aufgelöst werden, sodass das Polymer (6) im Inneren der aus den entgegengesetzt geladenen Polyelektrolyten (14a, 14b) gebildeten Hülle verkapselt ist (20).

Eine weitere Ausführungsform des erfindungsgemäßen Verfahrens ist in Figur 2 gezeigt. Es wird eine Suspension kolloidaler Templatpartikel (32) in einer Flüssigphase vorgelegt, die ein Polymer, z.B. eine Nukleinsäure, ein Protein, ein Polysaccharid oder ein synthetisches Polymer, in gelöster Form enthält. Durch Veränderung der Lösungsmittelzusammensetzung, z.B. tropfenweise Zugabe von Ethanol oder eines anderen Lösungsmittels, in dem das Polymer nicht oder nur schlecht löslich ist, erfolgt eine Präzipitation des Polymers, wobei mit dem Polymer beschichtete Templatpartikel (36) entstehen. Durch schichtweise Abscheidung von entgegengesetzt geladenen Polyelektrolyten (38a, 38b) werden beschichtete Templatpartikel mit anisotroper Hülle (40) erzeugt, wobei der innere Abschnitt der Hülle durch das präzipitierte Polymer und der äußere Abschnitt durch Schichten entgegengesetzt geladener Polyelektrolyten gebildet wird. Bei Verwendung löslicher Templatpartikel können diese aufgelöst werden, wobei ein in der Polyelektrolyt/Polyelektrolyt-Hülle verkapseltes Polymer (42) gebildet wird.

### Beispiele

### Beispiel 1 - Herstellung von PSS/PVBTAC-Kapselhüllen durch Einschrittpräzipitation

### 1.1 Materialien

Natriumpolystyrolsulfat mit einem Molekulargewicht von etwa 500.000 (PSS⁵⁰⁰) und Poly(vinylbenzyltrimethylammonium)chlorid mit einem Molekulargewicht von etwa 180.000 (PVBTAC) wurden von Polysiences Europe GmbH bezogen. Natriumpolystyrolsulfatmiteinem Molekulargewicht von etwa 70.000 (PSS⁷⁰) und Poly(allylaminhydrochlorid) mit einem Molekulargewicht von 50 bis 65.000 (PAH) wurden von Aldrich bezogen.

Teilvernetzte monodisperse Melamin-Formaldehyd-Partikel (MF-Latex) mit Durchmessern von 5,2 und 10 µm wurden von Microparticles GmbH, Berlin, Deutschland, bezogen. Diese Partikel sind in sauren Lösungen von HCl (pH ~ 1), Natriumpyrosulfitiösungen oder organischen Lösungsmitteln zersetzbar.

### 1.2 Methoden

### 1.2.1 Schichtweise Beschichtung (Vergleich)

Die Beschichtung von Templatpartikeln durch Membranfiltration erfolgte wie bei Voigt et al. (Ind. Eng. Chem. Res. 38 (1999), 4037) beschrieben. PSS⁵⁰⁰- oder PVBTAC-Adsorptions- (1 g/l in 0,5 M NaCl) und Wasch-Zyklen wurden in einer Membranfiltrationsvorrichtung (Millipore/Amicon Ultrafiltrationszelle 8200 und Millipore Membranfilter SSWP 090 25) abwechselnd durchgeführt. Aufgrund der negativen Ladung des MF-Latex wurde mit einer Adsorption von PSS⁵⁰⁰ begonnen. Nach Adsorption von zehn Schichten (jeweils fünf Schichten PSS⁵⁰⁰ und PVBTAC) wurden die beschichteten Teilchen gesammelt und in einem großen Volumen einer HCl-Lösung mit pH 1 suspendiert. Innerhalb weniger Sekunden wurde die Suspension transparent als Ergebnis einer Zersetzung der MF-Latex-Templatpartikel.

Nach weiteren Waschschritten durch Membranfiltration wurden schichtweise überzogene Mikrokapseln in anwendungsfertiger Form erhalten.

### 1.2.2 Oberflächenpräzipitation (Erfindung)

Das System PSS⁵⁰⁰ (3 g/l)/PVBTAC (1g/l) in Wasser (60 Gew.-%)/Aceton (20 Gew.-%)/Natriumbromid (20 Gew.-%) verhält sich gemäß dem von Michaels (Industrial Engineering Chemistry 57 (1965), 32) und Michaels et al. (J. Phys. Chem. 69 (1965), 1456), supra, veröffentlichten Phasendiagramm und ergibt eine klare Lösung ohne nachweisbare Turbidität. 5 ml dieses Systems wurden mit 1 ml gepackten 5,2 µm MF-Latexpartikel (positiv geladen) entsprechend etwa 0,85 m² Teilchenoberfläche versetzt. Das Lösungsfenster wurde auf zwei unterschiedliche Arten, nämlich durch langsame Verdampfung von Aceton bzw. durch langsame Zugabe von Wasser verlassen. Der Präzipitationsprozess wurde bei 20 ° C für eine Dauer von etwa 2 h durchgeführt. Dann wurde die Suspension gewonnen und weiter untersucht.

### 1.2.3 Rasterkraftmikroskopie (SFM)

SFM-Bilder wurden unter Verwendung eines Digital Instruments Nanoscope IIIa erhalten. Die Probe wurde durch Aufbringen eines Tropfens der Mikrokapselsuspension auf eine saubere Glimmeroberfläche und Trocknen an Luft hergestellt. Die getrockneten Mikrokapseln wurden im Kontaktmodus untersucht.

### 1.2.4 Konfokale Laserrastermikroskopie (CLSM)

Konfokale Bilder wurden mit dem konfokalen Laserrastermikroskop TCS SP von Leica unter Verwendung eines Aristoplan 100x Öl-Immersionsobjektivs erhalten. 10 µl der Suspension von beschichteten Teilchen wurden auf einen Objektträger gegeben. Zu dieser Suspension wurden 50 µl 0,1 Mol/l HCl gegeben. Nach 2 min wurden weitere 50 µl 0,1 Mol/l NaOH zugegeben. Geringe Mengen von Rhodamin 6G wurden als Fluoreszenzmarker für die Kapselwände zugesetzt.

### 1.3 Ergebnisse

Durch schichtweise Abscheidung gemäß dem Stand der Technik hergestellte Mikrokapseln zeigten eine typische ultradünne Schalenstruktur mit einer geringen Wanddicke von etwa 15 nm. Durch Zugabe des ternären Gemisches Wasser/Aceton/Natriumbromid konnten diese Mikrokapseln vollständig aufgelöst werden.

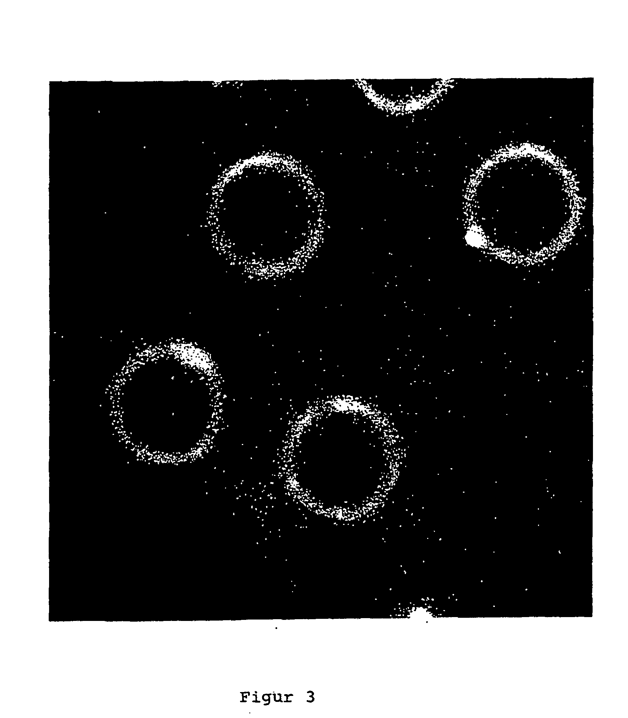

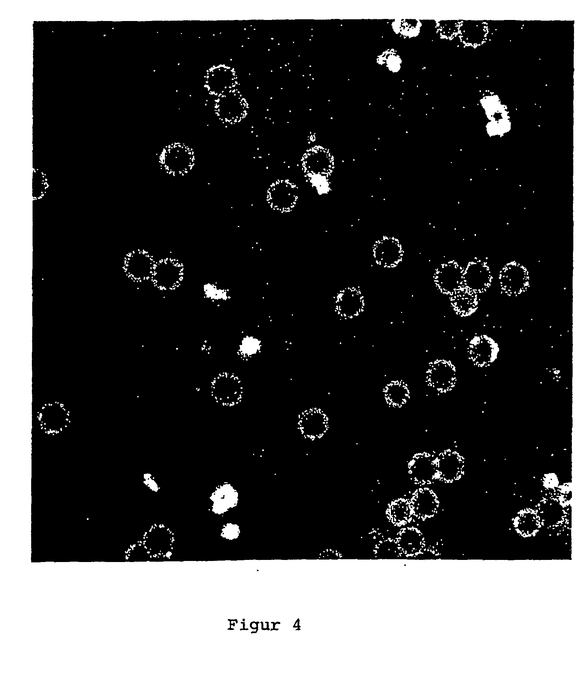

CLSM-Bilder von Kapseln, die durch die erfindungsgemäße Einschritt-Oberflächenpräzipitation hergestellt wurden, sind in den Figuren 3 und 4 gezeigt. In Figur 3 wurde das Lösungsfenster durch Acetonverdampfung und in Figur 4 durch Zugabe von Wasser verlassen. Größe und Form der Mikrokapseln ähneln denjenigen der Templatpartikel. Ein großer Anteil der Mikrokapseln ist etwas kleiner als die ursprünglichen Templatpartikel. Eine Untersuchung der Permeabilitätseigenschaften zeigte, dass - ebenso wie im Fall der schrittweise hergestellten Kapseln - kleine polare Farbstoffe die Hülle durchdringen können.

### Beispiel 2 - Hersteilung Polyelektrolyt-/lon- und Polymer-Kanselhüllen durch Einschritt-Präzipitation

### 2.1 Materialien

PSS mit einem Molekulargewicht von 70.000, PAH mit einem Molekulargewicht von 50.000 und Acridinorange (AO) wurden von Aldrich bezogen. Y(NO₃)₃, FeCl₃ und TbCl₃ wurden von Merck bezogen. Dipicolinsäure (DPA) und 4-Pyrensulfat (4-PS) wurden von Molecular probes erhalten. DNA und Dextran (Molekulargewicht 76.000) markiert mit Rhodamin (Rd) wurden von Sigma erworben.

Polystyrolatexpartikel (PS) modifiziert mit Sulfatgruppen (Durchmesser 468 nm) wurden wie bei Furizava et al. (Kolloid-Z. Z.Polym. 250 (1972), 908) beschrieben, hergestellt. Dispersionen von säurelöslichen Melaminformaldehydpartikeln (MF-Latex) mit Durchmessern von 4 und 6,5 µm wurden von Microparticels GmbH, Berlin, Deutschland bezogen.

### 2.2 Methoden

Konfokale Laserrastermikroskopie und Rasterkraftmikroskopie wurden wie in Beispiel 1 beschrieben, durchgeführt.

### 2.3 Ergebnisse

### 2.3.1 Präzipitation von Metallion/Polyelektrolyt-Hüllen

Eine Suspension der MF-Latexpartikel wurde mit Tb³⁺-lonen vermischt. Nach Zugabe des Polyanions PSS wurden Tb³⁺/PSS-Präzipitate gebildet. Die Suspension (1 ml) wurde kontinuierlich während des Zutropfens (10 µl) einer PSS-Rd-Lösung (1 mg/ml) gerührt, bis die PSS-Rd in Konzentration einen bestimmten Wert erreichte (Tabelle 1). Nach 10 bis 15 min wurden die Partikel abzentrifugiert und der Anteil an PSS-Rd-Molekülen, die nicht an die Partikel gebunden waren, durch Fluoreszenzbestimmung im Überstand bestimmt. Tabelle 1 zeigt die Daten bezüglich der Endkonzentration an MF-Teilchen, Tb³⁺-lonen und die Konzentration von PSS nach Zugabe zur Suspension. Bemerkenswerterweise wurden etwa 80 bis 85 % des eingesetzten PSS bei allen untersuchten Konzentrationen an die MF-Latexpartikel adsorbiert.

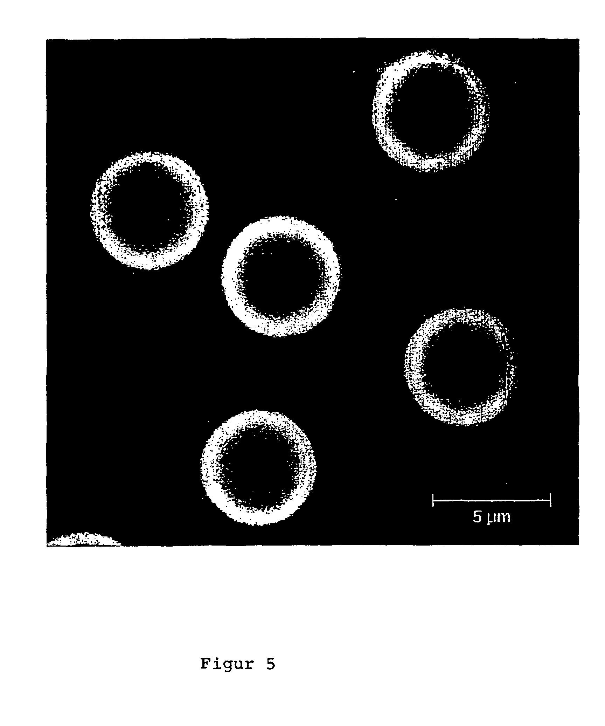

Die MF-Partikel wurden durch konfokale Mikroskopie untersucht. Ein typisches Bild für mit PSS/Tb³⁺ beschichtete MF-Partikel ist in Figur 5 gezeigt. Die Bedeckung der MF-Partikel mit Fluoreszenzmarkierung ist gleichmäßig. Außerhalb der Partikel wurde praktisch keine Fluoreszenzmarkierung gefunden.

**Tabelle 1**

| Experiment | MF-Partikel Konzentration | TbCl₃, M | PSS, monoM | Überstand Fluoreszenz, % | Geschätzte Menge an PSS-Monoschfchten |
|---|---|---|---|---|---|
| 1 | 10⁸ cm⁻³ | 10⁻³ | 2x10⁻⁴ | 10 | 20 |
| 2 | 10⁸ cm⁻³ | 10⁻³ | 10⁻³ | 15 | 80 |
| 3 | 10⁸ cm⁻³ | 3x10⁻³ | 3x10⁻³ | 13 | 250 |

### 2.3.2 Präzipitation von Polymerhüllen (Vergleichsbeispiel)

Die kontrollierte Präzipitation von Polymeren auf die Oberfläche von Kolloidpartikeln wurde durch Löslichkeitsverringerung von Polymeren durchgeführt. Als Polymere wurden DNA und Dextran aufgrund ihrer geringen Löslichkeit in Ethanol verwendet.

Zunächst wurden 1,5 ml einer MF-Latexpartiketsuspension (Teilchenkonzentration 5 x 10⁸/cm⁻³) mit einer DNA-Konzentration von 3 x 10¹⁴ Molekülen pro cm³ hergestellt. Dann wurde Ethanol tropfenweise zur Suspension bis zu einem Volumen von 4,5 ml gegeben. Während des Zutropfens von Ethanol wurde die Suspension geschüttelt. Nach 15 min wurde die Suspension zentrifugiert. Durch Bestimmung der Fluoreszenz (AO) im Überstand wurde gefunden, dass etwa 20 % der DNA nicht an die Partikel gebunden waren.

Ein entsprechendes Experiment wurde auch unter Verwendung von mit Rhodamin markiertem Dextran (Dextran-Rd) durchgeführt. Hierzu wurden 1 ml einer MF-Latexpartikelsuspension (Konzentration 5 x 10⁸ Partikel pro cm³) und Dextran-Rd (3 x 10¹⁵ Moleküle pro cm³) hergestellt. Nach der Präzipitation wurden etwa 5 % des Dextrans im Überstand gefunden.

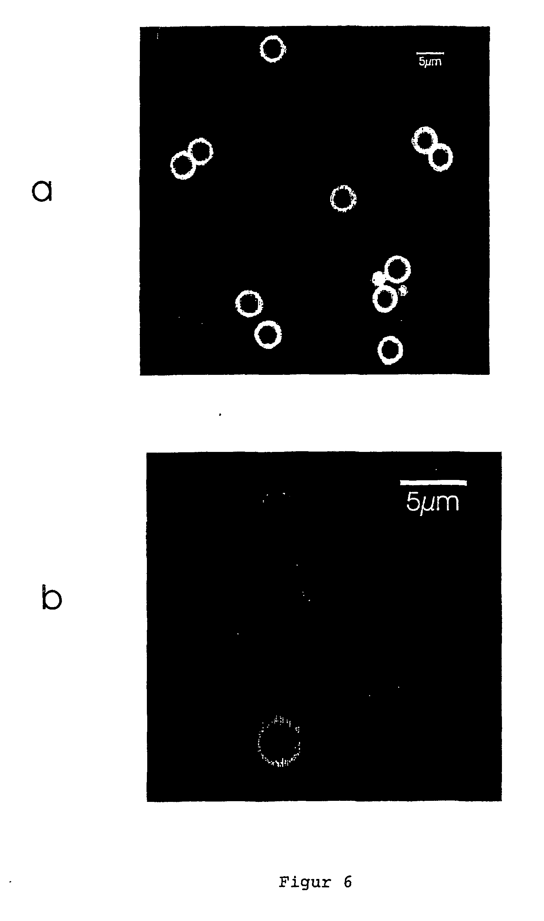

Typische Fluoreszenzkonfokalmikroskopbilder sind in den Figuren 6a und b dargestellt. Wie aus den Bildern ersichtlich ist, ist die Fluoreszenzmarkierung auf der Teilchenoberfläche homogen. Eine Abschätzung der mittleren Dicke des Polymerfilms auf dem Partikel ergibt für DNA einen Wert von etwa 50 monomolekularen Schichten an DNA, d.h. eine Dicke von etwa 100 nm.

### 2.3.3 Herstellung von leeren Hüllen aus Polyanion/Metallkomplexen

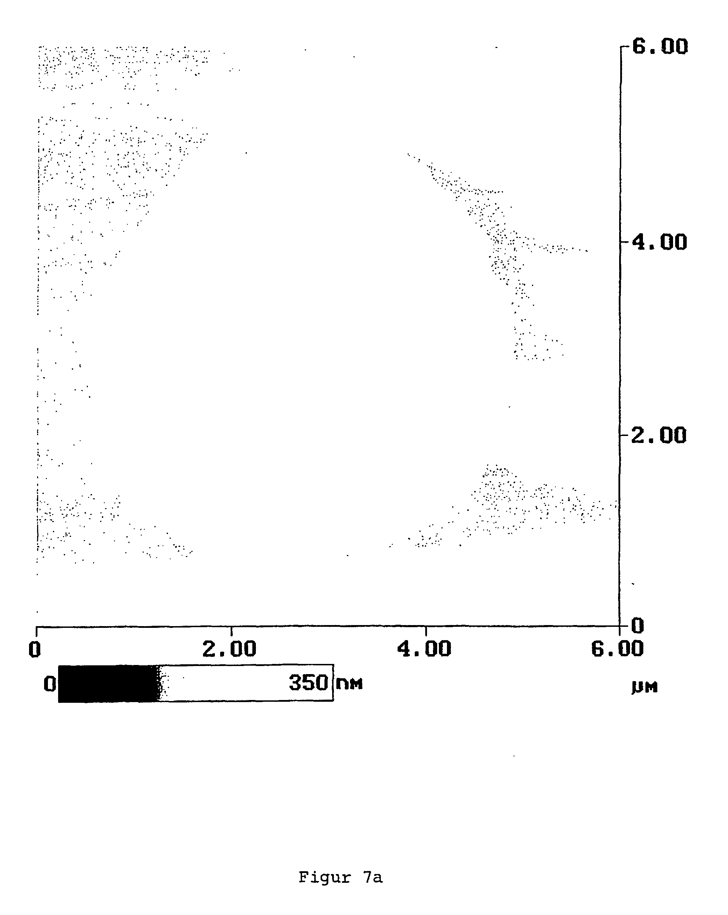

Die in 2.3.1 hergestellten Tb/PSS-beschichteten MF-Latexpartikel wurden mit 0,1 M HCl zersetzt. Die Proben wurden durch SFM untersucht. Figur 7a zeigt ein typisches Bild (Draufsicht) einer Kapsel mit 20 monomolekularen Tb/PSS-Schichten. Die in Lösung durch Konfokafmikroskopie beobachtete sphärische Form verändert sich nach dem Trocknen zu einer mehr polygonalen Gestalt. Die durchschnittliche minimale, aus mehreren Messungen erhaltene Höhe der Kapseln beträgt etwa 20 nm.

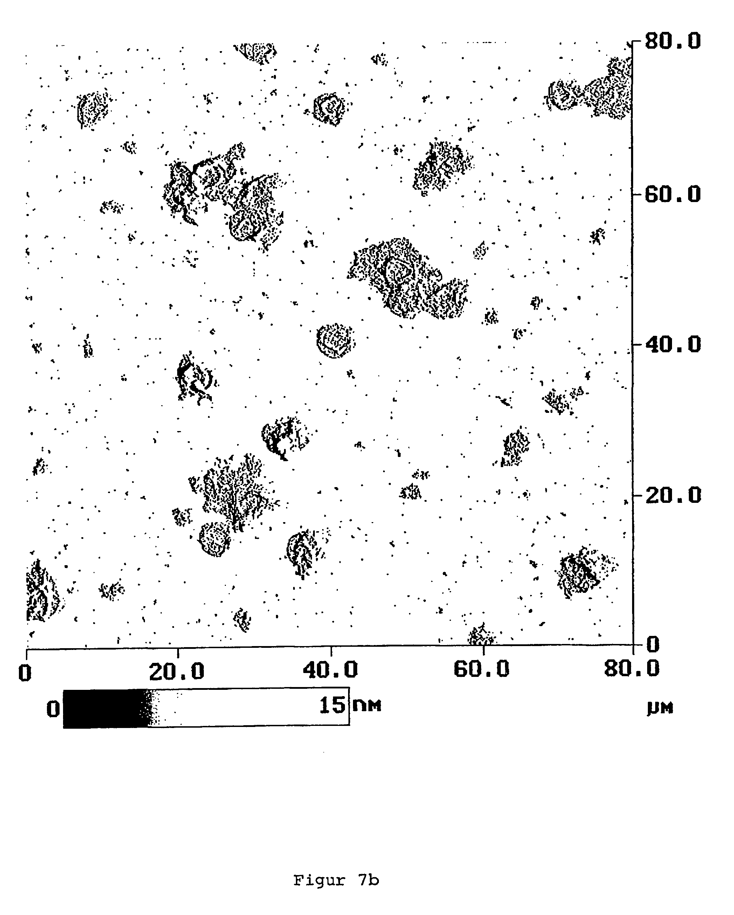

In Figur 7b ist die Draufsicht eines SFM-Bildes einer Probe mit mehreren Kapseln, bestehend aus etwa 100 monomolekularen Schichten von Tb/PSS gezeigt. Eine Anzahl der Kapseln ist zerbrochen. Es wird angenommen, dass diese geringere Stabilität auf die höhere Dicke der Hülle zurückzuführen ist, die die Permeabilität der Kapsel verringert. Dies führt beim Auflösen des MF-Latex zu einem höheren osmotischen Druck und somit zu einem leichteren Brechen der Kapseln.

### Beispiel 3 - Komplexe Präzipitation oder Koazervation aus alkalischer Lösung von PSS und PAH.

Es wurde eine Ausgangslösung der beiden Polyelektrolyte hergestellt, in der beide simultan ohne miteinander zu reagieren (ähnlich wie im ternären Lösungsmittel), in Lösung gehalten werden. Das wurde erreicht durch Vorlage von 10 ml 0,1 % (w/w) NaOH-Lösung mit 0,1 M NaCl. In dieser Lösung wurden nacheinander 15 mg PSS (MG 70.000) und 10 mg PAH (MG 50.000 bis 65.000) gelöst. Es wurde bis zur vollständigen Auflösung geschüttelt (ca. 15 Minuten). Diese Lösung ist anschließend für mehrere Stunden stabil. Es wurde 1 ml Melamin Formaldehyd (MF) Latex mit einem Durchmesser von z.B. 4,7 µm hinzugefügt. Anschließend wurde mit 1 % (w/w) HCl bis in den Neutralbereich titriert. Mikroskopische Kontrolle zeigt die Einkapselung der MF-Kerne. Nach Separation der gekapselten Teilchen von den Komplexen in der freien Lösung (z.B. Filtration, Zentrifugation, Sedimentation) führte die Auflösung der MF-Kerne in HCl-Lösung von pH 1 innerhalb kurzer Zeit (etwa einige Sekunden bis zu einigen Minuten) zu den gewünschten Mikrokapseln.

Durch die Zugabe der Säure wurden die Löslichkeitsbedingungen für beide Partner, separat betrachtet, verbessert. Ihre gegenseitige Anwesenheit führte dann allerdings zu weniger löslichen Komplexen.

### Beispiel 4 - Komplex-Präzipitation oder Koazervation aus alkalischer PSS/P-AH-Lösung mit Emulsionstropfen als Templat

Hierbei wird eine Ausgangslösung der beiden Polyelektrolyte hergestellt, in der beide simultan ohne miteinander zu reagieren in Lösung sind. Das wird erreicht durch Vorlage von 100 ml 0, 1 % (w/w) NaOH-Lösung mit 0,1 M NaCl. In dieser Lösung werden nacheinander 300 mg PSS (MG 70.000) und 200 mg PAH (MG 50-65.000) gelöst. Es wird bis zur vollständigen Auflösung geschüttelt. Diese Lösung ist für mehrere Stunden stabil. Es werden 20 ml Parfümöl dazugegeben. Mit dem Ultra-Turrax wird anschließend emulgiert und danach rasch mit 10 % (w/w) HCl bis in den Neutralbereich tritriert. Anschließend wird die Emulsion gereinigt, z.B. im Scheidetrichter mehrfach gewaschen. Es ergab sich eine über Monate stabile Emulsion.

### Beispiel 5 - Oberflächenpräzinitation aus einer Lösung enthaltend einen Komplex aus Polvelektrolyt und niedermolekularem Liganden und dem entsprechend entgegengesetzt geladenen Polyelektrolyten

Lösung I: 0,5 ml PAH-Lösung (MG 50.000 - 65.000, 1 mg/ml) mit NaCl (0,01 - 100 mM) + 750 µl Natriumsulfat-Lösung (10⁻² M); Lösung II: 0,5 ml PSS (MG 70.000, 5 mg/ml) + 10 µl Melamin Formaldehyd Templatpartikel mit 6,1 µm Durchmesser. Lösung II wird zu Lösung I gegeben und gerührt. Nach etwa 1 Stunde wird das System gereinigt (Separation der beschichteten Template von den freien Komplexen mittels Zentrifugation oder Filtration mit anschließenden Waschungen). Die Templatpartikel werden durch Überführen in HCl-Lösung von pH 1 aufgelöst und die Mikrokapseln werden durch weitere Reinigungsschritte gewonnen.

### Beispiel 6 - Oberflächenpräzipitation aus einer Lösung enthaltend partiell destabilisierte Potyelektrolvtkomplexe (Polykation/Polyanion) mittels Salzzugabe und/oder pH-Variation.

In 10 ml 1 M NaCl werden 20 mg PSS und 10 mg PAH eingebracht. Das System wird 10 Minuten gerührt. Anschließend wird 1 ml MF Latex von 4,7 µm zugegeben. Das System wird für mehrere Stunden gerührt. Anschließend wird gereinigt bzw. gewaschen mittels Zentrifugation oder Filtration und die Template in verdünnter HCl (pH ~ 1) aufgelöst und die Kapseln werden gewonnen.

In 10 ml Wasser werden 20 mg PAH und 10 mg PSS und nach der Komplexbildung 10 ml 1 M NaCl eingebracht. Dazu wird 1 ml MF Latex von 4,7 µm gegeben. Das System wird für mehrere Stunden gerührt. Anschließend wird gereinigt bzw. gewaschen mittels Zentrifugation oder Filtration und die Template in verdünnter HCl (pH ~ 1) aufgelöst und die Kapseln werden gewonnen.

### Beispiel 7 - Ein-Schritt-Präzipitation aus einer Lösung enthaltend einen Komplex aus einem Polyelektrolyten und einem mehrwertigen lon

Lösung I: 1 ml PSS-Lösung (2 mg/ml) wird mit 200 µl einer Y(NO₃)₃-Lösung (2 x 10⁻² M) gemischt. Das resultierende Ladungsverhältnis zwischen Sulfat und Yttrium ist 5:3.

Lösung II: 400 µl Öl werden mit 1 ml Wasser gemischt. Die Mischung wird für 3 bis 4 Minuten mit Ultraschall in einem Ultra-Turrax emulgiert.

Lösung l wird anschließend rasch zu Lösung II gegeben und die resultierende Emulsion für 2 Minuten im Vortex geschüttelt. Die Emulsion ist für mehr als 20 Stunden stabil und kann gegebenenfalls als Ausgangssystem für weitere Beschichtungen dienen.

Das erfindungsgemäße Verfahren ist universell anwendbar. Die physikochemischen Bedingungen des Mediums werden so eingestellt, z.B. durch hohen Salzgehalt, dass die präformierten oder/und sich frisch bildenden Polyelektrolytkomplexe in der Hüllflüssigkeit instabil sind. Es zeigt sich dann überraschenderweise, dass eine Verteilung der Polyelektrolyte auf alle beteiligten Compartments in endlicher Zeit, die man durch geeignete Parameter steuern kann, erfolgt. Dazu zählt natürlich auch die Phasengrenze Partikel/Medium oder Öl/Medium. Hier können sich die Polyelektrolyte in der bekannten dreidimensionalen Netzstruktur mit mehr oder weniger Wasser anordnen. Durch Nachbehandlung, z.B. in wässrigen Lösungen hoher Salzkonzentration, lässt sich diese in andere Konfigurationen überführen. Beispielsweise lässt sich eine ungenügend vernetzte Hülle in eine stärker vernetzte überführen. Es ergibt sich die Möglichkeit, sowohl Strukturen in der Nähe des thermodynamischen Gleichgewichtes zu erzeugen als auch solche, die Anpassungen an vorgegebene Ungleichgewichtssituationen bedeuten.

## Patentansprüche

1. Verfahren zum Aufbringen einer Hülle auf Templatpartikel umfassend die Schritte:
(a) Bereitstellen einer Dispersion von Templatpartike(n geeigneter Größe in einer salzhaltigen Flüssigphase, die zur Bildung der Hülle erforderliche Komponenten in gelöster Form enthält, und
(b) Präzipitieren der Komponenten aus der Flüssigphase auf die Templatpartikel unter solchen Bedingungen, dass eine Hülle mit einer Dicke von 1 bis 100 nm um die Templatpartikel erzeugt wird,
wobei die zur Bildung der Hülle erforderlichen Komponenten
(i) zwei entgegengesetzt geladene Polyelektrolyten,
(ii) ein polyvalentes niedermolekulares Kation und einen negativ geladenen Polyelektrolyten oder
(iii) ein polyvalentes niedermolekulares Anion und einen positiv geladenen Polyelektrolyten
umfassen.

2. Verfahren nach Anspruch 1,
**dadurch gekennzeichnet,**
**dass** die Templatpartikel ausgewählt werden aus festen, flüssigen, flüssig-kristallinen und gasförmigen Partikeln.

3. Verfahren nach Anspruch 1 oder 2,
**dadurch gekennzeichnet,**
**dass** die Templatpartikel aus Partikeln mit einem Durchmesser von bis zu 50 µm, insbesondere bis zu 10 µm ausgewählt werden.

4. Verfahren nach einem der Ansprüche 1 bis 3,
**dadurch gekennzeichnet,**
**dass** die Templatpartikel einen Wirkstoff enthalten.

5. Verfahren nach Anspruch 4,
**dadurch gekennzeichnet,**
**dass** der Wirkstoff ausgewählt wird aus Katalysatoren, insbesondere Enzymen, Nanopartikeln, pharmazeutischen Wirkstoffen, Sensormolekülen, Kristallen, Polymeren und Gasen.

6. Verfahren nach einem der Ansprüche 1 bis 5,
**dadurch gekennzeichnet,**
**dass** hohle Zellwandpartikel gewinnbar von Hefen oder anderen einzelligen oder mehrzelligen zellwandhaltigen Lebewesen oder hohle Pollenwandpartike! als Template verwendet werden.

7. Verfahren nach einem der Ansprüche 1 bis 3 oder 6,
**dadurch gekennzeichnet,**
**dass** als Templatpartikel lösliche Partiket verwendet werden.

8. Verfahren nach Anspruch 7,
**dadurch gekennzeichnet,**
**dass** als lösliche Partikel teilvernetzte Melamin-Formaldehyd-Partikel oder biologische Partikel wie etwa Zellen verwendet werden.

9. Verfahren nach einem der vorhergehenden Ansprüche,
**dadurch gekennzeichnet,**
**dass** die zur Bildung der Hülle erforderlichen Komponenten ein di- oder trivalentes niedermolekulares Kation und einen negativ geladenen Polyelektrolyten umfassen.

10. Verfahren nach einem der vorhergehenden Ansprüche,
**dadurch gekennzeichnet,**
**dass** die zur Bildung der Hülle erforderlichen Komponenten ein di- oder trivalentes niedermolekulares Anion und einen positiv geladenen Polyelektrolyten umfassen.

11. Verfahren nach einem der vorhergehenden Ansprüche,
**dadurch gekennzeichnet,**
**dass** die zur Bildung der Hülle erforderlichen Komponenten zumindest ein Makromolekül umfassen.

12. Verfahren nach Anspruch 11,
**dadurch gekennzeichnet,**
**dass** ein Biopolymer verwendet wird.

13. Verfahren nach Anspruch 11,
**dadurch gekennzeichnet,**
**dass** ein Gemisch aus makromolekularen und niedermolekularen biologischen Zellsubstanzen verwendet wird.

14. Verfahren nach Anspruch 11,
**dadurch gekennzeichnet,**
**dass** ein Gemisch aus makromolekularen und niedermolekularen abiogenen Substanzen verwendet wird.

15. Verfahren nach Anspruch 11,
**dadurch gekennzeichnet,**
**dass** ein Gemisch aus makromolekularen und niedermolekularen biogenen und abiogenen Substanzen verwendet wird.

16. Verfahren nach einem der Ansprüche 1 bis 15,
**dadurch gekennzeichnet,**
**dass** das Präzipitieren gemäß Schritt (b) die Zugabe einer Komponente der Hülle zur Flüssigphase umfasst.

17. Verfahren nach einem der Ansprüche 1 bis 15,
**dadurch gekennzeichnet,**
**dass** das Präzipitieren gemäß Schritt (b) eine die Präzipitation der Komponenten der Hülle bewirkende Veränderung der Flüssigphase umfasst.

18. Verfahren nach Anspruch 17,
**dadurch gekennzeichnet,**
**dass** die Veränderung der Flüssigphase eine Veränderung des pH-Werts oder/und eine Veränderung der Zusammensetzung der Flüssigphase umfasst.

19. Verfahren nach einem der Ansprüche 1 bis 15,
**dadurch gekennzeichnet,**
**dass** das Zusammenbringen der flüssigen salzhaltigen Hüllphase mit den Templaten die Systembedingungen derart verändert, dass ohne weitere äußere Stimulation mit Ausnahme der permanenten Durchmischung spontan der Aufbau von Hüllen erfolgt, die nach gegebenenfalls erfolgter Auflösung der Template intakt bleiben.

20. Verfahren nach einem der vorhergehenden Ansprüche, weiterhin umfassend die Durchführung von zumindest einem zusätzlichen Beschichtungsschritt vor oder/und nach dem Präzipitationsschritt.

21. Verfahren nach Anspruch 20,
**dadurch gekennzeichnet,**
**dass** der zusätzliche Beschichtungsschritt das Aufbringen einer Lipidschicht oder/und das schichtweise Aufbringen eines Polyelektrolyten umfasst.

22. Verfahren nach einem der vorhergehenden Ansprüche, weiterhin umfassend die Desintegration von löslichen Ternplatpartikeln.

23. Verfahren nach einem der vorhergehenden Ansprüche, weiterhin umfassend eine zumindest teilweise Desintegration der Hülle.

24. Verfahren nach einem der vorhergehenden Ansprüche,
**dadurch gekennzeichnet,**
**dass** eine Hülle mit einer Dicke von 1 bis 50 nm um die Templatpartikel erzeugt wird.

25. Verfahren nach einem der vorhergehenden Ansprüche,
**dadurch gekennzeichnet,**
**dass** eine Hülle mit einer Dicke von 5 bis 30 nm um die Templatpartikel erzeugt wird.

## Claims

1. Method for applying a shell to template particles comprising the steps:
(a) preparing a dispersion of template particles of suitable size in a salt-containing liquid phase which contains the components required to form the shell in a dissolved form and
(b) precipitating the components from the liquid phase onto the template particles under conditions which enable the formation of a shell around the template particles that has a thickness of from 1 to 100 nm,
wherein the components required to form the shell comprise
(i) two oppositely charged polyelectrolytes,
(ii) a polyvalent low-molecular cation and a negatively charged polyelectrolyte or
(iii) a polyvalent low-molecular anion and a positively charged polyelectrolyte.

2. Method as claimed in claim 1,
**characterized in that**
the template particles are selected from solid, liquid, liquid-crystalline and gaseous particles.

3. Method as claimed in claim 1 or 2,
**characterized in that**
the template particles are selected from particles having a diameter of up to 50 µm, in particular of up to 10 µm.

4. Method as claimed in one of the claims 1 to 3,
**characterized in that**
the template particles contain an active substance.

5. Method as claimed in claim 4,
**characterized in that**
the active substance is selected from catalysts, in particular enzymes, nanoparticles, pharmaceutical agents, sensor molecules, crystals, polymers and gases.

6. Method as claimed in one of the claims 1 to 5,
**characterized in that**
hollow cell wall particles obtainable from yeasts or other monocellular or multicellular living organisms containing cell walls or hollow pollen wall particles are used as templates.

7. Method as claimed in one of the claims 1 to 3 or 6,
**characterized in that**
soluble particles are used as template particles.

8. Method as claimed in claim 7,
**characterized in that**
partly cross-linked melamine-formaldehyde particles or biological particles such as cells are used as the soluble particles.

9. Method as claimed in one of the previous claims,
**characterized in that**
the components required to form the shell comprise a divalent or trivalent low-molecular cation and a negatively charged polyelectrolyte.

10. Method as claimed in one of the previous claims,
**characterized in that**
the components required to form the shell comprise a divalent or trivalent low-molecular anion and a positively charged polyelectrolyte.

11. Method as claimed in one of the previous claims,
**characterized in that**
the components required to form the shell comprise at least one macromolecule.

12. Method as claimed in claim 11,
**characterized in that**
a biopolymer is used.

13. Method as claimed in claim 11,
**characterized in that**
a mixture of macromolecular and low-molecular biological cell substances is used.

14. Method as claimed in claim 11,
**characterized in that**
a mixture of macromolecular and low-molecular abiogenic substances is used.

15. Method as claimed in claim 11,
**characterized in that**
a mixture of macromolecular and low-molecular biogenic and abiogenic substances is used.

16. Method as claimed in one of the claims 1 to 15,
**characterized in that**
the precipitation according to step (b) comprises adding a component of the shell to the liquid phase.

17. Method as claimed in one of the claims 1 to 15,
**characterized in that**
the precipitation according to step (b) comprises a change in the liquid phase which results in the precipitation of the components of the shell.

18. Method as claimed in claim 17,
**characterized in that**
the change in the liquid phase comprises a change of the pH value or/and a change in the composition of the liquid phase.

19. Method as claimed in one of the claims 1 to 15,
**characterized in that**
the system conditions are changed by mixing the liquid salt-containing shell phase with the templates in such a manner that the shells are formed spontaneously without further external stimulation with the exception of permanent mixing and remain intact after optional dissolution of the templates.

20. Method as claimed in one of the previous claims additionally comprising at least one additional coating step before or/and after the precipitation step.

21. Method as claimed in claim 20,
**characterized in that**
the additional coating step comprises the application of a lipid layer or/and the application of a polyelectrolyte in layers.

22. Method as claimed in one of the previous claims additionally comprising the disintegration of soluble template particles.

23. Method as claimed in one of the previous claims additionally comprising an at least partial disintegration of the shell.

24. Method as claimed in one of the previous claims,
**characterized in that**
a shell is formed around the template particles which has a thickness of 1 to 50 nm.

25. Method as claimed in one of the previous claims,
**characterized in that**
a shell is formed around the template particles which has a thickness of 5 to 30 nm.

## Revendications

1. Procédé pour appliquer une enveloppe sur des particules matrices comprenant les étapes :
(a) préparation d'une dispersion de particules matrices de taille appropriée dans une phase liquide contenant des sels, qui contient sous forme dissoute des composants nécessaires pour la formation de l'enveloppe, et
(b) précipitation des composants depuis la phase liquide sur les particules matrices dans des conditions telles qu'une enveloppe d'une épaisseur de 1 à 100 nm est produite autour des particules matrices,
où les composants nécessaires pour la formation de l'enveloppe comprennent
(i) deux polyélectrolytes chargés de manière opposée,
(ii) un cation de faible masse moléculaire plurivalent et un polyélectrolyte chargé négativement ou
(iii) un anion de faible masse moléculaire plurivalent et un polyélectrolyte chargé positivement.

2. Procédé selon la revendication 1 **caractérisé en ce que** les particules matrices sont choisies parmi les particules solides, liquides, cristallines liquides et gazeuses.

3. Procédé selon la revendication 1 ou 2 **caractérisé en ce que** les particules matrices sont choisies parmi les particules d'un diamètre pouvant atteindre 50 µm, en particulier 10 µm.

4. Procédé selon l'une des revendications 1 à 3 **caractérisé en ce que** les particules matrices contiennent un principe actif.

5. Procédé selon la revendication 4 **caractérisé en ce que** le principe actif est choisi parmi les catalyseurs, en particulier les enzymes, les nanoparticules, les principes actifs pharmaceutiques, les molécules capteurs, les cristaux, les polymères et les gaz.

6. Procédé selon l'une des revendications 1 à 5 **caractérisé en ce que** des particules de paroi cellulaire creuses pouvant être obtenues à partir de levures ou d'autres êtres vivants unicellulaires ou pluricellulaires contenant une paroi cellulaire ou des particules de paroi de pollen creuses sont utilisées comme matrices.

7. Procédé selon l'une des revendications 1 à 3 ou 6 **caractérisé en ce que** des particules solubles sont utilisées comme particules matrices.

8. Procédé selon la revendication 7 **caractérisé en ce que** des particules de mélamine-formaldéhyde partiellement réticulées ou des particules biologiques comme des cellules, par exemple, sont utilisées comme particules solubles.

9. Procédé selon l'une des revendications précédentes **caractérisé en ce que** les composants nécessaires pour la formation de l'enveloppe comprennent un cation de faible masse moléculaire di- ou trivalent et un polyélectrolyte chargé négativement.

10. Procédé selon l'une des revendications précédentes **caractérisé en ce que** les composants nécessaires pour la formation de l'enveloppe comprennent un anion de faible masse moléculaire di- ou trivalent et un polyélectrolyte. chargé positivement.

11. Procédé selon l'une des revendications précédentes **caractérisé en ce que** les composants nécessaires pour la formation de l'enveloppe comprennent au moins une macromolécule.

12. Procédé selon la revendication 11 **caractérisé en ce qu'**un biopolymère est utilisé.

13. Procédé selon la revendication 11 **caractérisé en ce qu'**un mélange de substances cellulaires biologiques macromoléculaires et de faible masse moléculaire est utilisé.

14. Procédé selon la revendication 11 **caractérisé en ce qu'**un mélange de substances abiogènes macromoléculaires et de faible masse moléculaire est utilisé.

15. Procédé selon la revendication 11 **caractérisé en ce qu'**un mélange de substances biogènes et abiogènes macromoléculaires et de faible masse moléculaire est utilisé.

16. Procédé selon l'une des revendications 1 à 15 **caractérisé en ce que** la précipitation selon l'étape (b) comprend l'addition d'un composant de l'enveloppe à la phase liquide.

17. Procédé selon l'une des revendications 1 à 15 **caractérisé en ce que** la précipitation selon l'étape (b) comprend une modification de la phase liquide provoquant la précipitation des composants de l'enveloppe.

18. Procédé selon la revendication 17 **caractérisé en ce que** la modification de la phase liquide comprend une modification du pH et/ou une modification de la composition de la phase liquide.

19. Procédé selon l'une des revendications 1 à 15 **caractérisé en ce que** la réunion de la phase d'enveloppe liquide contenant des sels avec les matrices modifie les conditions du système de telle manière que la formation d'enveloppes qui restent intactes après la dissolution éventuelle des matrices a lieu spontanément sans autre stimulation externe à l'exception du mélange permanent.

20. Procédé selon l'une des revendications précédentes comprenant en outre la mise en oeuvre d'au moins une étape de revêtement supplémentaire avant et/ou après l'étape de précipitation.

21. Procédé selon la revendication 20 **caractérisé en ce que** l'étape de revêtement supplémentaire comprend l'application d'une couche de lipides et/ou l'application par couches d'un polyélectrolyte.

22. Procédé selon l'une des revendications précédentes comprenant en outre la désintégration de particules matrices solubles.

23. Procédé selon l'une des revendications précédentes comprenant en outre une désintégration au moins partielle de l'enveloppe.

24. Procédé selon l'une des revendications précédentes **caractérisé en ce qu'**une enveloppe d'une épaisseur de 1 à 50 nm est produite autour des particules matrices.

25. Procédé selon l'une des revendications précédentes **caractérisé en ce qu'**une enveloppe d'une épaisseur de 5 à 30 nm est produite autour des particules matrices.
